# EUROPEAN PATENT APPLICATION

(11) **EP 4 130 048 A1**
(43) Date of publication of application: **08.02.2023**
(21) Application number: 21779289.4
(22) Date of filing: 16.03.2021
(51) Int. Cl.: C07K 19/00, C07K 1/22, C07K 1/18, C07K 1/16, C07K 1/36

(54) **METHOD FOR EFFICIENTLY SEPARATING AND PURIFYING RECOMBINANT HUMAN COAGULATE FACTOR VIII FC FUSION PROTEIN**

(30) Priority: 31.03.2020 CN 202010242530
(71) Applicant: Ampsource Biopharma Shanghai Inc., Shanghai 201318 (CN)
(72) Inventor: JIA, Shixiang, Shanghai 201318 (CN); XU, Shidong, Shanghai 201318 (CN); YU, Lingju, Shanghai 201318 (CN); ZHOU, Bo, Shanghai 201318 (CN); WU, Hong, Shanghai 201318 (CN); YUAN, Yonglong, Shanghai 201318 (CN); LI, Qiang, Shanghai 201318 (CN)
(74) Representative: HGF
(86) International application number: PCT/CN2021/081056
(87) International publication number: WO 2021/197051

(57) **Abstract**

Disclosed is a method for efficiently separating and purifying recombinant human coagulate factor VIII Fc fusion protein. The method comprises steps of affinity chromatography and anion exchange chromatography; and the sample captured by means of the affinity chromatography is eluted with a salt ion buffer containing 5%-20% polyol organic solvents under the condition of pH 4.0 to 8.0, and the protein sample can be separated and purified to 85% or more by further ProteinA affinity chromatography. The purification method is simple to operate, naturally connects each step of chromatography, has a high recovery rate and low cost, and easily increases production.

## Description

### TECHNICAL FIELD

The present application relates to the field of Fc fusion protein purification and, in particular, to a method for efficiently isolating and purifying recombinant human coagulation factor VIII Fc fusion protein.

### BACKGROUND

Coagulation Factor VIII (FVIII) is a multi structural macromolecular glycoprotein divided into six domains: three A domains (A1, A2 and A3), one carbohydrate-rich non-essential central domain (B domain) and two C domains (C1 and C2). A mature protein consists of light and heavy chains with a molecular weight of approximately 280 kDa. A light chain has a molecular weight of about 80 kDa and includes A3, C1 and C2 in structure, which are connected in the following manner: A3-C1-C2. A heavy chain has a molecular weight of about 90 to 200 kDa and includes A1, A2 and B in structure, which are connected in the following manner: A1-A2-B. There is a metal ion-dependent link between the heavy chain and the light chain. In plasma, the dimer consisting of the heavy chain and the light chain then binds to von Willebrand factor (VWF) with high affinity to be protected from being degraded prior to maturation.

At present, the purification process of coagulation Factor VIII has the problems of a complex process, a high cost and low purity (Stefan Winge et al., Protein Expression and Purification, 2015, 115:165-175). The effective removal of impurities through an affinity chromatography process is a key factor in downstream protein preparation. However, at present, the purification step in the affinity chromatography of FVIII mostly has the problems of a low recovery rate and a relatively high cost, and the elution step in the affinity chromatography uses a large amount of expensive reagents such as ethylene glycol, propylene glycol and arginine, which shortens the service life of a filler, leads to more organic solvent residues, reduces the recovery rate, and greatly increases the purification cost. Although GE Corporation has developed a new purification filler, VIII select (Justin T. McCue et al., Journal of Chromatography A, 2009, 1216:7824-7830), there are still problems such as a relatively high filler cost, a low recovery rate and expensive reagents used in an eluent. At present, the purification process of long-acting recombinant human coagulation factor VIII developed by the manufacturers of INDs or marketed long-acting human coagulation factor VIII drugs, such as Fu Jen and Biogen, still has the problems of a complicated operation, a high cost and a low recovery rate.

There are a large number of patients with hemophilia A worldwide, and many patients have not been diagnosed or registered. The market demand is far greater than the production capacity, and recombinant human coagulation factor VIII Fc fusion protein cannot be widely used due to a high price. The production cost is the main factor of the high price of recombinant protein drugs. Thus, there is an urgent need in the art to develop a new method for purifying recombinant human coagulation factor VIII Fc fusion protein, which is efficient, convenient, low in cost and suitable for industrial application.

### SUMMARY

An object of the present application is to establish a method for purifying recombinant human coagulation factor VIII Fc fusion protein, which is simple to operate and has a high recovery rate and a low production cost.

The present application provides a method for isolating or purifying recombinant human coagulation factor VIII Fc fusion protein (simply referred to as fusion protein). The method includes the following steps:
(1) affinity chromatography: capturing the fusion protein with an affinity medium, protein A, then eluting the fusion protein with an elution buffer, and collecting an isolated product;
(2) anion-exchange chromatography: loading the above isolated product onto a strong anion-exchange chromatography medium of a quaternary ammonium salt to isolate structural variants and polymers and remove contaminants such as host cell proteins (HCPs) and DNA, performing gradient elution with an elution buffer, and collecting an isolated product;
wherein, the fusion protein is a fusion of natural human FVIII or human FVIII with domain B deleted/truncated and a variant thereof with a constant region sequence (Fc domain) of any human antibody isotype or a variant thereof via a peptide linker; the Fc domain is selected from, for example, heavy chain constant regions of human IgG1, IgG2, IgG3, IgG4, IgM, IgA1, IgA2, IgD and IgE or variants thereof; more preferably selected from heavy chain constant regions of human IgG1, IgG2, IgG3 and IgG4 or variants thereof; and in a preferred embodiment of the present application, an amino acid sequence of the recombinant human coagulation factor VIII Fc fusion protein is as shown in SEQ ID NO: 2.

Preferably, the affinity chromatography is performed on an affinity medium selected from the group consisting of MabSelect, MabSelect SuRe, Protein A Diamond and MabSelect SuRe LX; preferably, MabSelect SuRe LX is used.

Preferably, in the affinity chromatography, before eluted, the fusion protein is washed with a decontamination buffer containing no organic solvent or containing an organic solvent with a relatively low concentration to remove part of contaminants; where the decontamination buffer includes 20 mM His-HCl, 0.4-1.5 M NaCl, 10 mM CaCl₂, 0.02-0.05% Tween-80 and 0-10% ethylene glycol and has a pH of 5.0 to 7.0; preferably, the decontamination buffer includes 20 mM His-HCl, 1.5 M NaCl, 10 mM CaCl₂, 0.02% Tween-80 and 3-7% ethylene glycol and has a pH of 5.0 to 7.0.

The elution buffer used in the affinity chromatography includes one or more salts, one or more polyol organic solvents and one or more surfactants with a concentration of 0.2 M to 1.5 M; the elution buffer further contains calcium ions with a concentration of 5 mM to 20 mM; the elution buffer further contains a His-HCL buffer of 10 mM to 50 mM; and the elution buffer has a pH of 4.0 to 8.0, preferably 5.0 to 6.0, and more preferably 5.0.

Preferably, examples of the salts include sodium chloride, ammonium chloride, potassium chloride, sodium sulfate, ammonium sulfate, etc; the preferred salt in the present application is a chloride; and the most preferred salt in the present application is sodium chloride.

Preferably, the polyol organic solvent includes, but is not limited to, ethylene glycol, 1,2-propanediol and glycerol, preferably ethylene glycol; and a proportion of the organic solvent is 5-40% (w/w), preferably 5-20%.

Preferably, the calcium ions are selected from soluble calcium salts, including, but not limited to, calcium chloride, calcium acetate, calcium lactate and calcium benzoate, preferably calcium chloride.

Preferably, the surfactant is Tween-80 or Triton X-100 and has a concentration of 0.01% to 0.1% (v/v), preferably 0.02% to 0.05% (v/v).

Preferably, the elution buffer includes 20 mM His-HCl, 0.5-2.0 M NaCl, 5-20 mM CaCl₂, 0.02-0. 1% Tween-80 and 2-20% ethylene glycol, and the elution buffer has a pH of 4.5 to 7.0.

In a preferred embodiment of the present application, the affinity chromatography specifically includes the following steps:
(1) equilibrating a chromatography column with 3-5 column volumes of an equilibration buffer (20 mM His-HCl, 0.2 M NaCl, 10 mM CaCl₂, 0.02% Tween-80, with a pH of 6.8 to 7.2);
(2) loading the sample with a load not higher than 50000 IU/mL;
(3) after loading, equilibrating the chromatography column with 3-5 column volumes of the above equilibration buffer and washing unbound components;
(4) washing the chromatography column with 3-5 column volumes of a decontamination buffer (20 mM His-HCl, 0.5 M NaCl, 10 mM CaCl₂, 0.02% Tween-80, with a pH of 7.0) to remove part of contaminants; and
(5) eluting the fusion protein with the elution buffer (20 mM His-HCl, 1.5 M NaCl, 10 mM CaCl₂, 0.02% Tween-80, 15% ethylene glycol, with a pH of 5.0) and collecting target peaks.

Preferably, the anion-exchange chromatography is performed on a strong anion-exchange medium selected from the group consisting of Q HP, Toyopearl GigaCap Q-650, DEAE Beads 6FF, Generik MC-Q, Fractogel EMD TMAE and Q Ceramic HyperD F; preferably, Q HP is used.

In a preferred embodiment of the present application, the anion-exchange chromatography specifically includes the following steps:
(1) diluting an eluted product of the affinity chromatography by a factor of 3-10 with a diluent (20 mM His-HCl, 10 mM NaCl, 10 mM CaCl₂, 0.02% Tween-80, with a pH of 7.0);
(2) equilibrating a chromatography column with 3-5 column volumes of an equilibration buffer (20 mM His-HCl, 100 mM NaCl, 10 mM CaCl₂, 0.02% Tween-80, with a pH of 7.0 to 7.5);
(3) loading the sample with a load of 5000-50000 IU/mL;
(4) after loading, washing the chromatography column with 3-5 column volumes of the above equilibration buffer;
(5) washing the chromatography column with 3-5 column volumes of an elution buffer (20 mM His-HCl, 500 mM NaCl, 10 mM CaCl₂, 0.02% Tween-80, with a pH of 7.0 to 7.5) to remove part of protein impurities;
(6) performing gradient elution with 3-5 column volumes of each salt ion elution buffer (20 mM His-HCl, 1 M NaCl, 10 mM CaClz, 0.02% Tween-80, with a pH of 7.0 to 7.5) ranging from 50% to 70% and collecting an eluted sample.

Preferably, the method for isolating or purifying recombinant human coagulation factor VIII Fc fusion protein (simply referred to as fusion protein) in the present application further includes hydrophobic chromatography in a flow-through mode. Specific steps are described as follows:
(1) affinity chromatography: capturing the fusion protein with an affinity medium, protein A, eluting the fusion protein with an elution buffer, and collecting an isolated product;
(2) anion-exchange chromatography: loading the above isolated product onto a strong anion-exchange chromatography medium of a quaternary ammonium salt to isolate structural variants and remove contaminants such as HCPs and DNA, performing gradient elution, and collecting an isolated product;
(3) hydrophobic chromatography: loading the above isolated product onto a weakly hydrophobic medium containing four or eight carbons, washing a chromatography column with an equilibration buffer to bind impurities and polymers in the above isolated product to a hydrophobic chromatography medium, directly flowing through the fusion protein, and collecting an isolated product;
or, the hydrophobic chromatography in the flow-through mode precedes the anion-exchange chromatography, and specific steps are described as follows:
(1) affinity chromatography: capturing the fusion protein with an affinity medium, protein A, eluting the fusion protein with an elution buffer, and collecting an isolated product;
(2) hydrophobic chromatography: loading the above isolated product onto a weakly hydrophobic medium containing four or eight carbons, washing a chromatography column with an equilibration buffer to bind impurities and polymers in the above isolated product to a hydrophobic chromatography medium, directly flowing through the fusion protein, and collecting an isolated product;
(3) anion-exchange chromatography: loading the above isolated product onto a strong anion-exchange chromatography medium of a quaternary ammonium salt to isolate structural variants and remove contaminants such as HCPs and DNA, performing gradient elution, and collecting an isolated product;
wherein, the fusion protein is a fusion of natural human FVIII or human FVIII with domain B deleted/truncated and a variant thereof with a constant region sequence (Fc domain) of any human antibody isotype or a variant thereof via a peptide linker; the Fc domain is selected from, for example, heavy chain constant regions of human IgG1, IgG2, IgG3, IgG4, IgM, IgA1, IgA2, IgD and IgE or variants thereof; more preferably selected from heavy chain constant regions of human IgG1, IgG2, IgG3 and IgG4 or variants thereof; and in a preferred embodiment of the present application, an amino acid sequence of the recombinant human coagulation factor VIII Fc fusion protein is as shown in SEQ ID NO: 2.

Preferably, the affinity chromatography is performed on an affinity medium selected from the group consisting of MabSelect, MabSelect SuRe, Protein A Diamond and MabSelect SuRe LX; preferably, MabSelect SuRe LX is used.

The elution buffer used in the affinity chromatography includes one or more salts, one or more polyol organic solvents and one or more surfactants with a concentration of 0.2 M to 1.5 M; the elution buffer further contains calcium ions with a concentration of 5 mM to 20 mM; the elution buffer further contains a His-HCL buffer of 10 mM to 50 mM; and the elution buffer has a pH of 4.0 to 8.0, preferably 5.0 to 6.0, and more preferably 5.0.

Preferably, examples of the salts include sodium chloride, ammonium chloride, potassium chloride, sodium sulfate, ammonium sulfate, etc; the preferred salt in the present application is a chloride; and the most preferred salt in the present application is sodium chloride.

Preferably, the polyol organic solvent includes, but is not limited to, ethylene glycol, 1,2-propanediol and glycerol, preferably ethylene glycol; and a proportion of the organic solvent is 5-40% (w/w), preferably 5-20%.

Preferably, the calcium ions are selected from soluble calcium salts, including, but not limited to, calcium chloride, calcium acetate, calcium lactate and calcium benzoate, preferably calcium chloride.

Preferably, the surfactant is Tween-80 or Triton X-100 and has a concentration of 0.01% to 0.1% (v/v), preferably 0.02% to 0.05% (v/v).

Preferably, the elution buffer includes 20 mM His-HCl, 0.5-2.0 M NaCl, 5-20 mM CaClz, 0.02% Tween-80 and 5-20% ethylene glycol, and the elution buffer has a pH of 5.0 to 7.0.

Preferably, in the affinity chromatography, before eluted, the fusion protein is washed with a decontamination buffer containing no organic solvent or containing an organic solvent with a relatively low concentration to remove part of contaminants; where the decontamination buffer includes 20 mM His-HCl, 0.4-1.5 M NaCl, 10 mM CaClz, 0.02% Tween-80 and 0-10% ethylene glycol and has a pH of 5.0 to 7.0; preferably, the decontamination buffer includes 20 mM His-HCl, 1.5 M NaCl, 10 mM CaClz, 0.02% Tween-80 and 3-7% ethylene glycol and has a pH of 5.0 to 7.0.

In a preferred embodiment of the present application, the affinity chromatography specifically includes the following steps:
(1) equilibrating a chromatography column with 3-5 column volumes of an equilibration buffer (20 mM His-HCl, 0.2 M NaCl, 10 mM CaCl₂, 0.02% Tween-80, with a pH of 6.8 to 7.2);
(2) loading the sample with a load not higher than 50000 IU/mL;
(3) after loading, equilibrating the chromatography column with 3-5 column volumes of the above equilibration buffer and washing unbound components;
(4) washing the chromatography column with 3-5 column volumes of a decontamination buffer (20 mM His-HCl, 0.5 M NaCl, 10 mM CaClz, 0.02% Tween-80, with a pH of 7.0) to remove part of contaminants;
(5) eluting the fusion protein with the elution buffer (20 mM His-HCl, 1.5 M NaCl, 10 mM CaClz, 0.02% Tween-80, 15% ethylene glycol, with a pH of 5.0) and collecting target peaks.

Preferably, the hydrophobic chromatography is performed on a weakly hydrophobic medium selected from the group consisting of Capto Octyl, Octyl Sepharose 4FF and Phenyl Sepharose 6FF; preferably, Capto Octyl is used;
wherein, the equilibration buffer used in the hydrophobic chromatography in includes 20 mM His-HCl, 0.1-1.0 M NaCl, 10 mM CaClz, 0.02% Tween-80 and 0-10% ethylene glycol and has a pH of 6.8 to 7.2.

In a preferred embodiment of the present application, the hydrophobic chromatography specifically includes the following steps:
(1) diluting an eluted product of the affinity chromatography with a diluent (20 mM His-HCl, 10 mM NaCl, 10 mM CaCl₂, 0.02% Tween-80, with a pH of 7.0);
(2) equilibrating a chromatography column with 3-5 column volumes of an equilibration buffer (20 mM His-HCl, 0.1-1 M NaCl, 10 mM CaCl₂, 0.02% Tween-80, 0-8% ethylene glycol, with a pH of 6.8 to 7.2);
(3) loading the sample with a load of less than 100000 IU/mL;
(4) after loading, washing the chromatography column with 3-5 column volumes of the above equilibration buffer and collecting a flow-through product.

Preferably, the anion-exchange chromatography is performed on a strong anion-exchange medium selected from the group consisting of Q HP, Toyopearl GigaCap Q-650, DEAE Beads 6FF, Generik MC-Q, Fractogel EMD TMAE and Q Ceramic HyperD F; preferably, Q HP is used.

In a preferred embodiment of the present application, the anion-exchange chromatography specifically includes the following steps:
(1) diluting the flow-through product of the hydrophobic chromatography with a diluent (20 mM His-HCl, 10 mM NaCl, 10 mM CaCl₂, 0.02% Tween-80, with a pH of 7.0);
(2) equilibrating a chromatography column with 3-5 column volumes of an equilibration buffer (20 mM His-HCl, 100 mM NaCl, 10 mM CaCl₂, 0.02% Tween-80, with a pH of 7.0 to 7.5);
(3) loading the sample with a load of 5000-10000 IU/mL;
(4) after loading, washing the chromatography column with 3-5 column volumes of the above equilibration buffer;
(5) washing the chromatography column with 3-5 column volumes of an elution buffer (20 mM His-HCl, 500 mM NaCl, 10 mM CaCl₂, 0.02% Tween-80, with a pH of 7.0 to 7.5) to remove part of protein impurities;
(6) performing gradient elution with 3-5 column volumes of each salt ion elution buffer (20 mM His-HCl, 1 M NaCl, 10 mM CaClz, 0.02% Tween-80, with a pH of 7.0 to 7.5) ranging from 50% to 70% and collecting an eluted sample.

The method for isolating or purifying recombinant human coagulation factor VIII Fc fusion protein in the present application has the advantages described below.
1. The purification cost is greatly reduced, most expensive reagents (for example, arginine) are removed, and the types and amounts of organic solvents are greatly reduced so that the amount of organic reagent residues is greatly reduced. In this manner, not only detection steps are reduced but also the filler is reused much more times, and it is easier to meet the requirement on the limit of organic solvent residues in the drug registration.
2. Host cell proteins (HCPs) and product-related impurities are effectively removed in the affinity chromatography step. For example, high molecular weight (HMW) and low molecular weight (LMW) substances are key factors affecting a downstream protein purification process, and protein purity after the "capturing" significantly affects the number and type of subsequent purification steps. After one-step purification through the affinity chromatography in the method provided by the present application, the protein purity can reach more than 85% so that the content of polymers is greatly reduced, which greatly reduces the difficulty of downstream purification and simplifies the subsequent purification process (for example, molecular sieve chromatography can be omitted), and greatly improves a total recovery rate.
3. The hydrophobic chromatography is performed in the flow-through mode instead of an adsorption-elution mode so that the use of resins can be greatly reduced, the purity can be improved to about 95%, and the recovery rate can reach 60-80%.
4. The sample is less treated between different purification steps and the process is easy to industrialize for simple operation and smooth transition between steps. For example, the flow-through sample of the hydrophobic chromatography can be directly loaded for the anion-exchange chromatography without any treatment.
5. The process produces no pollution and waste water and is more environmentally friendly.
6. The purity of the purified sample can reach more than 95% and the overall recovery rate can reach about 25% so that the production cost is reduced and the process is easy to industrialize.

### BRIEF DESCRIPTION OF DRAWINGS

FIG. 1 shows an SEC-HPLC detection result of an eluted sample of affinity chromatography.
FIG. 2 shows an SEC-HPLC detection result of a flow-through sample of hydrophobic chromatography.
FIG. 3 shows an SEC-HPLC detection result of an eluted sample of ion-exchange chromatography.
FIG. 4 shows an SEC-HPLC detection result of a purified sample transferred into another solution.

### DEFINITION

"Factor VIII" or "FVIII" as used herein refers to a human plasma glycoprotein that is a member of an endogenous coagulation pathway and essential for coagulation. "Natural FVIII" is a full-length human FVIII molecule as shown in SEQ ID NO: 1 (amino acids 1-2332), where its domain B corresponds to the sequence shown by amino acids 741-1648 in SEQ ID NO: 1.

The factor VIII molecule of the present application may be an FVIII molecule with domain B deleted/truncated and a variant thereof, wherein the remaining domains substantially correspond to the sequences shown by amino acids 1-740 and 1649-2332 in SEQ ID NO: 1. In some aspects, the FVIII molecule with domain B deleted/truncated in the present application is a variant obtained by introducing amino acid modifications (substitutions, additions or deletions) at some positions, which means that the remaining domains (i.e., three A domains and two C domains) may have, for example, 1-20 or more amino acid substitutions, deletions or additions relative to the amino acid sequence (amino acids 1-740 and 1649-2332) corresponding to SEQ ID NO: 1 or are approximately 1%, 2%, 3%, 4%, 5%, 10%, 15% or 20% different. The modified FVIII variant with domain B deleted/truncated has FVIII activity which is at least 40%, at least 50%, at least 60%, at least 70%, at least 80%, at least 90%, 100% or even more than 100% of the activity of natural human FVIII.

"Fc fusion protein" or "Fc fusion derivative" is meant herein to include an FVIII derivative of the present application fused with an Fc domain that may be derived from any antibody isotype.

The Fc domain may also be modified to modulate some effector functions, such as complement binding and/or binding to some Fc receptors. The fusion of FVIII with an Fc domain having an ability to bind to an FcRn receptor generally causes the circulating half-life of the fusion protein to be longer (than the half-life of natural FVIII).

The term "elution buffer" refers to a conventional meaning, i.e., a buffer having appropriate pH and/or ionic strength or a buffer, to release one or more proteins from an isolation matrix. As used herein, the "salt" in the elution step refers to an alkaline-earth metal, an alkali metal salt or an ammonium salt, i.e. a salt having inorganic or organic cations and from an alkaline-earth metal, an alkali metal element or an ammonium cation. Examples of such salts include sodium chloride, ammonium chloride, sodium sulfate, ammonium sulfate, etc. The preferred salt in the present application is a chloride or sulfate; and the most preferred salt in the present application is sodium chloride.

The term "buffer" includes those reagents that can maintain the pH of a solution within a desired range. In the context of the present application, the desired range refers to a range of 4.0 to 8.0, for example, 5.0. The concentration range of the buffer is selected to maintain the preferred pH of the solution. The buffer may also be a mixture of at least two buffers, wherein the mixture can provide a pH within a specified range. The buffer may include, but is not limited to, histidine (L-histidine) and TRIS. In one embodiment, the buffer used is 20 mM histidine.

### DETAILED DESCRIPTION

To facilitate understanding, the present application is described in detail below through specific drawings and examples. It is to be particularly noted that the description is merely illustrative and not to limit the scope of the present application. Many changes and alterations of the present application based on the discussion in the specification are apparent to those skilled in the art.

### Example 1 Purification of recombinant human coagulation factor VIII Fc fusion protein by two-step chromatography

The amino acid sequence of recombinant human coagulation factor VIII Fc fusion protein isolated or purified in this example is as shown in SEQ ID NO: 2 in the sequence listing of the present application, i.e., the fusion protein as shown in SEQ ID NO: 9 and as described in CN201811123918.X is expressed by Chinese hamster ovary (CHO) cells. For the method for constructing an expression vector, reference is made to International Application No. CN201811123918.X. The fermentation broth obtained by the above method is subjected to centrifugation or depth filtration to obtain a supernatant used for the subsequent isolation or purification of the fusion protein.

The recombinant human coagulation factor VIII protein was purified by two-step chromatography in this example. The two-step chromatography included affinity chromatography and anion-exchange chromatography (The protein purifier used in this example was AKTA pure 25 M from GE, U.S. Reagents used in this example were purchased from Sinopharm Chemical Reagent Co., Ltd and had purity at an analytical grade).

In a first step, affinity chromatography was performed. Sample capture and concentration and the removal of part of contaminants were performed using Mabselect Sure from GE or other commercially available affinity media. Firstly, chromatography columns were equilibrated with 3-5 column volumes (CVs) of an equilibration buffer (20 mM His-HCl, 0.2 M NaCl, 10 mM CaCl₂, 0.02% Tween-80, pH 6.8-7.2) at a linear flowrate of 50-200 cm/h. The clarified fermentation broth was loaded at a linear flowrate of 50-200 cm/h with a load not higher than 50000 IU/mL. After loading, the chromatography columns were equilibrated with 3-5 column volumes (CVs) of an equilibration buffer (20 mM His-HCl, 0.2 M NaCl, 10 mM CaCl₂, 0.02% Tween-80, pH 6.8-7.2) at a linear flowrate of 50-200 cm/h to remove unbound components. The chromatography columns were washed with 3-5 column volumes of a decontamination buffer (20 mM His-HCl, 0.5 M NaCl, 10 mM CaCl₂, 0.02% Tween-80, pH 7.0) at a linear flowrate of 50-200 cm/h to remove part of contaminants. The target product was eluted using an elution buffer (20 mM His-HCl, 1.5 M NaCl, 10 mM CaCl₂, 0.02% Tween-80, 15% ethylene glycol, pH 5.0) at a linear flowrate of 50-200 cm/h and target peaks were collected. It was detected that the purity of the sample was up to 90% and the recovery rate was up to 75%.

In a second step, anion-exchange chromatography was performed. Fine purification was performed by using Q-HP from Bestchrom Company or other commercially available anion-exchange chromatography media to isolate structural variants and further remove contaminants such as HCPs and DNA. Firstly, chromatography columns were washed with 3-5 column volumes (CVs) of an equilibration buffer (20 mM His-HCl, 100 mM NaCl, 10 mM CaClz, 0.02% Tween-80, pH 7.0-7.5) at a linear flowrate of 50-200 cm/h. The load was controlled to be 5000-50000 IU/mL. Before loading, the sample was diluted by a factor of 3-10 with a diluent (20 mM His-HCl, 10 mM NaCl, 10 mM CaCl₂, 0.02% Tween-80, pH 7.0). After loading, the chromatography columns were washed with 3-5 column volumes (CVs) of an equilibration buffer (20 mM His-HCl, 100 mM NaCl, 10 mM CaCl₂, 0.02% Tween-80, pH 7.0-7.5) at a linear flowrate of 50-200 cm/h. Then, the chromatography columns were washed with 3-5 column volumes (CVs) of an elution buffer (20 mM His-HCl, 500 mM NaCl, 10 mM CaClz, 0.02% Tween-80, pH 7.0-7.5) at a linear flowrate of 50-200 cm/h to remove part of protein impurities. Finally, gradient elution was performed with 3-5 column volumes (CVs) of an elution buffer (20 mM His-HCl, 1 M NaCl, 10 mM CaCl₂, 0.02% Tween-80, pH 7.0-7.5) with salt concentration gradients of 50% to 70% at a linear flowrate not higher than 200 cm/h. The eluted sample was collected and sent for the detection of protein content, SEC-HPLC, activity and HCP content, separately. The purity was up to 95% and the recovery rate was up to 70%. The remaining samples were immediately transferred into a preparation solution using G25 or UF/DF for preservation.

### Example 2 Purification of recombinant human coagulation factor VIII Fc fusion protein by three-step chromatography

The recombinant human coagulation factor VIII Fc fusion protein isolated or purified in this example was from the same source as that in Example 1. The recombinant human coagulation factor VIII Fc fusion protein was purified by three-step chromatography in this example. The three-step chromatography included affinity chromatography, hydrophobic chromatography and anion-exchange chromatography (The protein purifier used in this example was AKTA pure 25 M from GE, U.S. Reagents used in this example were purchased from Sinopharm Chemical Reagent Co., Ltd and had purity at an analytical grade).

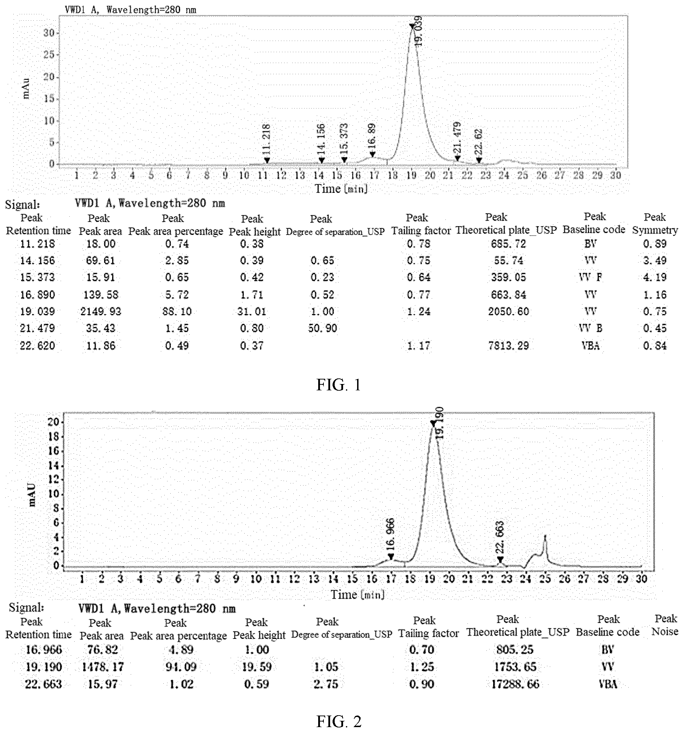

In a first step, affinity chromatography was performed. Sample capture and concentration and the removal of part of contaminants were performed using Mabselect Sure from GE or other commercially available affinity media. Firstly, chromatography columns were equilibrated with 3-5 column volumes (CVs) of an equilibration buffer (20 mM His-HCl, 0.2 M NaCl, 10 mM CaCl₂, 0.02% Tween-80, pH 6.8-7.2) at a linear flowrate of 50-200 cm/h. The clarified fermentation broth was loaded at a linear flowrate of 50-200 cm/h with a load not higher than 50000 IU/mL. After loading, the chromatography columns were equilibrated with 3-5 column volumes (CVs) of an equilibration buffer (20 mM His-HCl, 0.2 M NaCl, 10 mM CaCl₂, 0.02% Tween-80, pH 6.8-7.2) at a linear flowrate of 50-200 cm/h to remove unbound components. The chromatography columns were washed with 3-5 column volumes of a decontamination buffer (20 mM His-HCl, 0.5 M NaCl, 10 mM CaCl₂, 0.02% Tween-80, pH 7.0) at a linear flowrate of 50-200 cm/h to remove part of contaminants. The target product was eluted using an elution buffer (20 mM His-HCl, 1.5 M NaCl, 10 mM CaCl₂, 0.02% Tween-80, 15% ethylene glycol, pH 5.0) at a linear flowrate of 50-200 cm/h and target peaks were collected and detected. The SEC-HPLC detection result of the eluted product of the affinity chromatography is shown in FIG. 1. It was detected that the purity of the sample was up to 88% and the recovery rate was up to 70%.

In a second step, hydrophobic chromatography was performed. Intermediate purification was performed using Capto Octyl from GE or other commercially available hydrophobic chromatography media to reduce the content of polymers. The object was to remove part of polymers to make the content of polymers lower than 5%. Firstly, affinity samples were diluted by a factor of 2 with a diluent (20 mM His-HCl, 10 mM NaCl, 10 mM CaCl₂, 0.02% Tween-80, pH 7.0). Chromatography columns were equilibrated with 3-5 column volumes (CVs) of an equilibration buffer (20 mM His-HCl, 0.1-1 M NaCl, 10 mM CaCl₂, 0-8% ethylene glycol, 0.02% Tween-80, pH 6.8-7.2) at a linear flowrate of 50-200 cm/h. The load was controlled to be lower than 100000 IU/mL. After loading, the chromatography columns were washed with 3-5 column volumes (CVs) of the equilibration buffer at a linear flowrate of 50-200 cm/h. The sample was collected and sent for the detection of SEC-HPLC, activity and electrophoresis. The SEC-HPLC detection result of the flow-through product of the hydrophobic chromatography is shown in FIG. 2. The purity was up to 94% and the recovery rate was 85%.

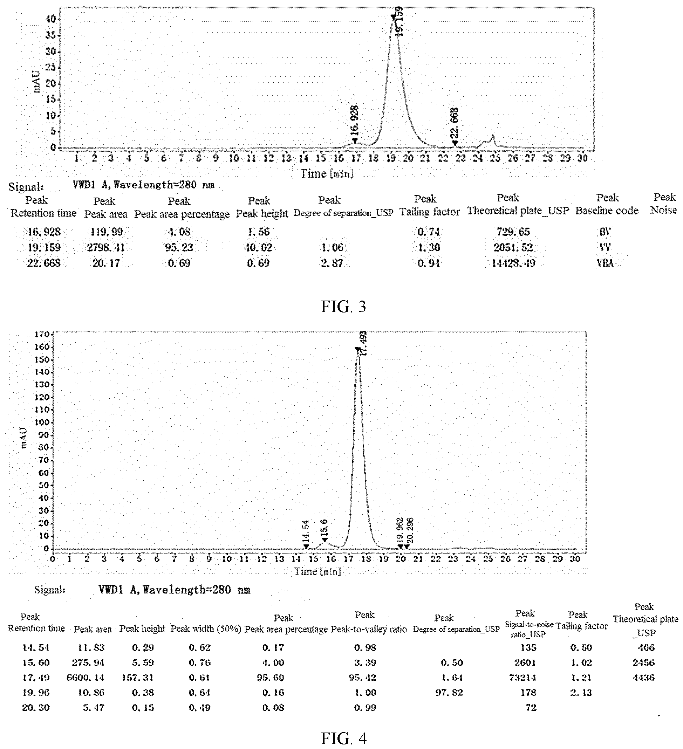

In a third step, anion-exchange chromatography was performed. Fine purification was performed by using Q-HP from Bestchrom Company or other commercially available anion-exchange chromatography media to isolate structural variants and further remove contaminants such as HCPs and DNA. Firstly, chromatography columns were washed with 3-5 column volumes (CVs) of an equilibration buffer (20 mM His-HCl, 100 mM NaCl, 10 mM CaCl₂, 0.02% Tween-80, pH 7.0-7.5) at a linear flowrate of 50-200 cm/h. The load was controlled to be 5000-10000 IU/mL. Before loading, the sample was diluted by a factor of 0-3 with a diluent (20 mM His-HCl, 10 mM NaCl, 10 mM CaCl₂, 0.02% Tween-80, pH 7.0). The chromatography columns were washed with 3-5 column volumes (CVs) of an equilibration buffer (20 mM His-HCl, 100 mM NaCl, 10 mM CaCl₂, 0.02% Tween-80, pH 7.0-7.5) at a linear flowrate of 50-200 cm/h. Then, the chromatography columns were washed with 3-5 column volumes (CVs) of an elution buffer (20 mM His-HCl, 500 mM NaCl, 10 mM CaCl₂, 0.02% Tween-80, pH 7.0-7.5) at a linear flowrate of 50-200 cm/h to remove part of protein impurities. Finally, gradient elution was performed with 3-5 column volumes (CVs) of an elution buffer (20 mM His-HCl, 1 M NaCl, 10 mM CaCl₂, 0.02% Tween-80, pH 7.0-7.5) with salt concentration gradients of 50% to 70% at a linear flowrate not higher than 200 cm/h. The eluted sample was collected and sent for the detection of protein content, SEC-HPLC, activity and HCP content, separately. The SEC-HPLC detection result of the eluted product of the anion-exchange chromatography is shown in FIG. 3. The purity was up to 95% and the recovery rate was 70%. The remaining samples were immediately transferred into a preparation solution using G25 or UF/DF for preservation. The SEC-HPLC detection result of the product transferred into another solution is shown in FIG. 4.

In some embodiments, the anion-exchange chromatography follows the affinity chromatography, which is followed by the hydrophobic chromatography in a flow-through mode.

All the publications mentioned in the present application are incorporated herein by reference as if each publication is separately incorporated herein by reference. In addition, it should be understood that those skilled in the art who have read the disclosure can make various changes or modifications to the present application, and these equivalent forms fall within the scope of the appended claims.

## Claims

1. A method for isolating or purifying recombinant human coagulation factor VIII (FVIII) Fc fusion protein, comprising the following steps:
(1) affinity chromatography: capturing the fusion protein with an affinity medium, protein A, eluting the fusion protein with an elution buffer, and collecting an isolated product;
(2) anion-exchange chromatography: loading the isolated product in the above step onto a strong anion-exchange chromatography medium of a quaternary ammonium salt, performing gradient elution with an elution buffer, and collecting the fusion protein;
wherein, the fusion protein is a fusion of natural human FVIII or human FVIII with domain B deleted/truncated and a variant thereof with a constant region sequence (Fc domain) of any human antibody isotype or a variant thereof via a peptide linker; the Fc domain is selected from, for example, heavy chain constant regions of human IgG1, IgG2, IgG3, IgG4, IgM, IgA1, IgA2, IgD and IgE or variants thereof; more preferably selected from heavy chain constant regions of human IgG1, IgG2, IgG3 and IgG4 or variants thereof; and in a preferred embodiment of the present application, an amino acid sequence of the recombinant human coagulation factor VIII Fc fusion protein is as shown in SEQ ID NO: 2;
wherein, the elution buffer used in the affinity chromatography comprises one or more salts, one or more polyol organic solvents and one or more surfactants with a concentration of 0.2 M to 1.5 M; the elution buffer further contains calcium ions with a concentration of 5 mM to 20 mM; the elution buffer further contains a His-HCL buffer of 10 mM to 50 mM; and the elution buffer has a pH of 4.0 to 8.0, preferably 5.0 to 6.0, and more preferably 5.0.

2. The method of claim 1, wherein the affinity chromatography is performed on an affinity medium selected from the group consisting of MabSelect, MabSelect SuRe, Protein A Diamond and MabSelect SuRe LX; preferably, MabSelect SuRe LX is used.

3. The method of claim 1, wherein the anion-exchange chromatography is performed on a strong anion-exchange medium selected from the group consisting of Q HP, Toyopearl GigaCap Q-650, DEAE Beads 6FF, Generik MC-Q, Fractogel EMD TMAE and Q Ceramic HyperD F; preferably, Q HP is used.

4. The method of claim 1, wherein in the affinity chromatography, before eluted, the fusion protein is washed with a decontamination buffer containing no organic solvent or containing an organic solvent with a relatively low concentration to remove part of contaminants; wherein the decontamination buffer comprises 20 mM His-HCl, 0.4-1.5 M NaCl, 10 mM CaCl₂, 0.02-0.05% Tween-80 and 0-10% ethylene glycol and has a pH of 5.0 to 7.0; preferably, the decontamination buffer comprises 20 mM His-HCl, 1.5 M NaCl, 10 mM CaCl₂, 0.02% Tween-80 and 3-7% ethylene glycol and has a pH of 5.0 to 7.0.

5. The method of claim 1, wherein the salt comprised in the elution buffer used in the affinity chromatography comprises sodium chloride, ammonium chloride, potassium chloride, sodium sulfate, ammonium sulfate, etc.; preferably chlorides; most preferably, sodium chloride.

6. The method of claim 1, wherein the polyol organic solvent comprised in the elution buffer used in the affinity chromatography comprises ethylene glycol, 1,2-propanediol and glycerol, preferably ethylene glycol; and a proportion of the organic solvent is 5-40% (w/w), preferably 5-20%.

7. The method of claim 1, wherein the calcium ions comprised in the elution buffer used in the affinity chromatography are selected from soluble calcium salts comprising calcium chloride, calcium acetate, calcium lactate and calcium benzoate; preferably calcium chloride.

8. The method of claim 1, wherein the surfactant comprised in the elution buffer used in the affinity chromatography is Tween-80 or Triton X-100 and has a concentration of 0.01% to 0.1% (v/v), preferably 0.02% to 0.05% (v/v).

9. The method of claim 1, wherein the elution buffer used in the affinity chromatography comprises 20 mM His-HCl, 0.5-2.0 M NaCl, 5-20 mM CaCl₂, 0.02-0.1% (v/v) Tween-80 and 2-20% (w/w) ethylene glycol, and the elution buffer has a pH of 4.5 to 7.0.

10. The method of claim 1, wherein the affinity chromatography specifically comprises the following steps:
(1) equilibrating a chromatography column with 3-5 column volumes of an equilibration buffer (20 mM His-HCl, 0.2 M NaCl, 10 mM CaCl₂, 0.02% Tween-80, with a pH of 6.8 to 7.2);
(2) loading the sample with a load not higher than 50000 IU/mL;
(3) after loading, equilibrating the chromatography column with 3-5 column volumes of the above equilibration buffer and washing unbound components;
(4) washing the chromatography column with 3-5 column volumes of a decontamination buffer (20 mM His-HCl, 0.5 M NaCl, 10 mM CaCl₂, 0.02% Tween-80, with a pH of 7.0) to remove part of contaminants;
(5) eluting the fusion protein with the elution buffer (20 mM His-HCl, 1.5 M NaCl, 10 mM CaCl₂, 0.02% Tween-80, 15% ethylene glycol, with a pH of 5.0) and collecting target protein peaks.

11. The method of claim 1, wherein the anion-exchange chromatography specifically comprises the following steps:
(1) diluting an eluted product of the affinity chromatography with a diluent (20 mM His-HCl, 10 mM NaCl, 10 mM CaCl₂, 0.02% Tween-80, with a pH of 7.0);
(2) equilibrating a chromatography column with 3-5 column volumes of an equilibration buffer (20 mM His-HCl, 100 mM NaCl, 10 mM CaCl₂, 0.02% Tween-80, with a pH of 7.0 to 7.5);
(3) loading the sample with a load of 5000-50000 IU/mL;
(4) after loading, washing the chromatography column with 3-5 column volumes of the above equilibration buffer;
(5) washing the chromatography column with 3-5 column volumes of an elution buffer (20 mM His-HCl, 500 mM NaCl, 10 mM CaCl₂, 0.02% Tween-80, with a pH of 7.0 to 7.5) to remove part of protein impurities;
(6) performing gradient elution with 3-5 column volumes of each salt ion elution buffer (20 mM His-HCl, 1 M NaCl, 10 mM CaCl₂, 0.02% Tween-80, with a pH of 7.0 to 7.5) ranging from 50% to 70% and collecting an eluted sample.

12. The method of any one of claims 1 to 11, wherein the method further comprises hydrophobic chromatography in a flow-through mode, wherein the hydrophobic chromatography comprises: loading the isolated product of the affinity chromatography or the isolated product of the anion-exchange chromatography onto a weakly hydrophobic medium containing four or eight carbons, washing a chromatography column with an equilibration buffer to bind impurities and polymers in the above product to a hydrophobic chromatography medium, directly flowing through the fusion protein, and collecting an isolated product.

13. The method of claim 12, wherein the hydrophobic chromatography is performed on a weakly hydrophobic medium selected from the group consisting of Capto Octyl, Octyl Sepharose 4FF and Phenyl Sepharose 6FF; preferably, Capto Octyl is used.

14. The method of claim 12, wherein the equilibration buffer used in the hydrophobic chromatography comprises 20 mM His-HCl, 0.1-1.0 M NaCl, 10 mM CaCl₂, 0.02% Tween-80 and 0-10% ethylene glycol and has a pH of 6.8 to 7.2.

15. The method of claim 12, wherein the affinity chromatography specifically comprises the following steps:
(1) equilibrating a chromatography column with 3-5 column volumes of an equilibration buffer (20 mM His-HCl, 0.2 M NaCl, 10 mM CaCl₂, 0.02% Tween-80, with a pH of 6.8 to 7.2);
(2) loading the sample with a load not higher than 50000 IU/mL;
(3) after loading, equilibrating the chromatography column with 3-5 column volumes of the above equilibration buffer and washing unbound components;
(4) washing the chromatography column with 3-5 column volumes of a decontamination buffer (20 mM His-HCl, 0.5 M NaCl, 10 mM CaCl₂, 0.02% Tween-80, with a pH of 7.0) to remove part of contaminants;
(5) eluting the fusion protein with the elution buffer (20 mM His-HCl, 1.5 M NaCl, 10 mM CaCl₂, 0.02% Tween-80, 15% ethylene glycol, with a pH of 5.0) and collecting target peaks.

16. The method of claim 12, wherein the hydrophobic chromatography specifically comprises the following steps:
(1) diluting an eluted product of the affinity chromatography with a diluent (20 mM His-HCl, 10 mM NaCl, 10 mM CaCl₂, 0.02% Tween-80, with a pH of 7.0);
(2) equilibrating a chromatography column with 3-5 column volumes of an equilibration buffer (20 mM His-HCl, 0.1-1 M NaCl, 10 mM CaCl₂, 0.02% Tween-80, 0-8% ethylene glycol, with a pH of 6.8 to 7.2);
(3) loading the sample with a load of less than 100000 IU/mL;
(4) after loading, washing the chromatography column with 3-5 column volumes of the above equilibration buffer and collecting a flow-through product.

17. The method of claim 12, wherein the anion-exchange chromatography specifically comprises the following steps:
(1) diluting a flow-through product of the hydrophobic chromatography with a diluent (20 mM His-HCl, 10 mM NaCl, 10 mM CaCl₂, 0.02% Tween-80, with a pH of 7.0);
(2) equilibrating a chromatography column with 3-5 column volumes of an equilibration buffer (20 mM His-HCl, 100 mM NaCl, 10 mM CaCl₂, 0.02% Tween-80, with a pH of 7.0 to 7.5);
(3) loading the sample with a load of 5000-10000 IU/mL;
(4) after loading, equilibrating the chromatography column with 3-5 column volumes of the above equilibration buffer;
(5) washing the chromatography column with 3-5 column volumes of an elution buffer (20 mM His-HCl, 500 mM NaCl, 10 mM CaClz, 0.02% Tween-80, with a pH of 7.0 to 7.5) to remove part of protein impurities;
(6) performing gradient elution with 3-5 column volumes of each salt ion elution buffer (20 mM His-HCl, 1 M NaCl, 10 mM CaClz, 0.02% Tween-80, with a pH of 7.0 to 7.5) ranging from 50% to 70% and collecting an eluted sample.
